# EUROPEAN PATENT SPECIFICATION

(11) **EP 4 729 103 B1**
(45) Date of publication and mention of the grant of the patent: **11.11.2026**
(21) Application number: 25162971.3
(22) Date of filing: 11.03.2025
(51) Int. Cl.: A61M 25/01, A61N 1/00, A61M 25/00, A61M 25/10

(54) **BALLOON CATHETER WITH RETRACTABLE INNER SUPPORT**
BALLONKATHETER MIT RÜCKZIEHBARER INNENSTÜTZE
CATHÉTER À BALLONNET AVEC SUPPORT INTERNE RÉTRACTABLE

(30) Priority: 21.10.2024 KR 20240143849 U
(43) Date of publication of application: 22.04.2026
(60) Divisional application: 26202952.3 / 4 825 429
(73) Proprietor: RADEXEL INC., Chuncheon-si, Gangwon-do 24437 (KR)
(72) Inventor: JUNG, Nuri Hyun, 24363 Chuncheon-si (KR); BAHNG, Jungbae, 30147 Sejong-si (KR); KIM, Seongjun, 04759 Seoul (KR); KIM, Tae Hoon, 03469 Seoul (KR)
(74) Representative: HGF

(56) References cited:
- KR-A- 20130 009 445
- US-A1- 20250 065 086
- US-B1- 9 028 401

## Description

### [TECHNICAL FIELD]

The present disclosure relates to a balloon catheter.

### [BACKGROUND ART]

Radiation therapy is to irradiate a lesion of a living body with radiation such as X-rays, gamma rays, electron rays, and proton rays to delay or destroy the growth of the lesion tissue of the living body. Here, the lesion tissue may be cancer, and the like.

During radiation therapy, the distribution of the radiation dose may change depending on the density of the medium located in the path through which the radiation beam penetrates the living body. Specifically, if two media with different densities exist in the path through which the radiation penetrates the living body, the distribution of the radiation dose in the boundary area of the two media may be greater than in other areas.

During radiation therapy, to reduce the radiation effect on the surrounding tissue of the lesion tissue of the living body, a balloon may be inserted into the body cavity (hereinafter referred to as the "target site") around the lesion tissue of the living body to fix the lesion tissue and the target site of the living body. Here, the balloon may be inserted into the target site of the living body through an internal catheter (or guide wire). Specifically, for example, the internal catheter may be inserted into the inside of the balloon, and then the balloon and the internal catheter may be inserted into the target site of the living body. At this time, the internal catheter inside the balloon caused the following problems.

First, the internal catheter inside the balloon made it impossible to lower the internal density of the balloon below a certain level. For reference, increasing the density inside the balloon is easy to achieve by simply injecting a high-density material into the balloon.

In addition, the internal catheter inside the balloon causes interference with particle beams during radiation therapy using particles such as electrons, protons, and carbon ions, which changes the point of arrival of the particle beams.

Furthermore, the internal catheter inside the balloon changes its position inside the balloon, causing uncertainty in the radiation dose distribution inside and around the balloon.

In addition, the internal catheter inside the balloon causes secondary electrons to be generated inside the balloon when performing MCRT (Magnetic Controlled Radiation Therapy) that controls the path of radiation using a magnetic field during radiation therapy such as X-rays, reducing the effect of radiation dose control.

Therefore, it is necessary to eject the internal catheter inside the balloon while the balloon is inserted into the target area of the living body. In this case, the general method of ejecting the internal catheter is to eject the internal catheter from inside the balloon while the balloon is not inflated. However, this method has the following problems.

First, there is a problem that the position of the balloon changes due to various directional forces from the internal organs and muscles inside the living body at the moment the internal catheter is ejected from inside the balloon while the balloon is inserted into the target area of the living body.

In addition, when there is pressure inside the living body, there is a problem that the balloon get stuck in the internal catheter at the moment the internal catheter is ejected from inside the balloon while the balloon is inserted into the target area of the living body.

It is important for radiation therapy to maintain the position of the balloon at the target area of the living body. Therefore, the conventional method of ejecting the internal catheter from inside the balloon while the balloon is not inflated is difficult to apply due to changes in the position of the balloon, and as a result, the balloon capable of ejecting the internal catheter has not been used during radiation therapy.

### [Prior art document]

(Patent Document 1) Korea Patent Registration No. 10-2013-0009445 (Published on January 23, 2013)
US 9028401B1 relates to balloon systems configured to access and dilate body lumen and cavities.

### [DETAILED DESCRIPTION OF THE INVENTION]

### [TECHINICAL PROBLEM]

The present disclosure is to provide a balloon catheter that can improve the accuracy of radiation therapy for lesion tissue and target area of a living body by allowing an internal catheter inserted into the balloon to be discharged while the balloon is inflated and fixes the lesion tissue and target area of the living body.

The problems to be solved by the present disclosure are not limited to the problems mentioned above, and other problems that are not mentioned will be clearly understood by those skilled in the art from the description below.

### [TECHNICAL SOLUTION]

The present invention is defined by the independent claims. Further aspects of the present invention are outlined in the dependent claims. According to an embodiment of the present disclosure, a balloon catheter includes an external catheter; a balloon connected to one side of the external catheter and capable of being inflated or contracted and inserted into a target site of a living body; an internal catheter having one side inserted into the balloon and the other side penetrating into an inside of the external catheter and capable of moving in a first direction inserted into the balloon or a second direction discharged from the balloon; and a driving part configured to move and discharge the internal catheter inserted into the inside of the balloon in the second direction in a state that the balloon is inflated and fixed to the target site of the living body.

Furthermore, the external catheter includes a coupling part coupled to an outer circumference of the internal catheter; a stretchable part coupled to the coupling part and capable of being stretched; and a guide part coupled to the stretchable part and configured to guide a movement of the internal catheter, and wherein the balloon is connected to the guide part.

Furthermore, the stretchable part may have a bellows shape.

Furthermore, the balloon catheter includes a first stopper configured to limit a movement range of the internal catheter in the second direction.

Furthermore, the first stopper includes a hook groove formed in the external catheter; and a hook formed in the internal catheter and hooked to the hook groove when the internal catheter moves in the second direction.

Furthermore, the balloon catheter includes a second stopper configured to limit a movement range of the internal catheter in the first direction and a movement range of the second direction.

Furthermore, the second stopper includes two catch protrusions spaced apart with a spacing and connected along a movement direction of the internal catheter to the outer circumference of the external catheter; and a moving protrusion connected to the outer circumference of the internal catheter and is arranged between the two catch protrusions.

Furthermore, the driving part may include an actuator configured to move the internal catheter in the second direction.

Furthermore, the driving part may include a fluid supply part configured to inject fluid into the balloon so that the internal catheter moves in the second direction by the fluid injected into the balloon.

Furthermore, the balloon catheter includes a third stopper detachably fixed to the internal catheter and configured to limit a movement range of the internal catheter in the first direction.

Furthermore, the third stopper may have a U-shape.

Other specific details of the present disclosure are included in the detailed description and drawings.

### [ADVANTAGEOUS EFFECTS OF THE INVENTION]

According to one embodiment of the present disclosure, the balloon catheter can discharge the internal catheter inserted inside the balloon while the balloon is inflated and fixes lesion tissue and target area of a living body, thereby improving the accuracy of radiation therapy for the lesion tissue and target area of the living body during radiation therapy.

The effects of the present disclosure are not limited to the effects mentioned above, and other effects not mentioned will be clearly understood by those skilled in the art from the description below.

### [DESCRIPTION OF THE DRAWINGS]

FIG. 1 is a cross-sectional view illustrating a balloon catheter according to one embodiment of the present disclosure.
FIG. 2 is a cross-sectional view illustrating the balloon of FIG. 1 in an inflated state.
FIG. 3 is a cross-sectional view illustrating a first stopper of a balloon catheter according to an embodiment of the present disclosure.
FIG. 4 is a cross-sectional view illustrating the balloon of FIG. 3 in an inflated state.
FIG. 5 is a cross-sectional view illustrating a second stopper of a balloon catheter according to one embodiment of the present disclosure.
FIG. 6 is a cross-sectional view illustrating the balloon of FIG. 5 in an inflated state.
FIGS. 7 to 10 are cross-sectional views illustrating an operation process of a balloon catheter according to another embodiment of the present disclosure.
FIG. 11 is a schematic diagram illustrating a state in which a balloon of a balloon catheter according to one embodiment of the present disclosure is fixed to a target site of a living body.
FIG. 12 is a schematic diagram illustrating a state in which a balloon of a conventional balloon catheter is fixed to a target site of a living body.
FIG. 13 is a cross-sectional diagram illustrating a process of radiation therapy of a target site of a living body using a balloon catheter according to one embodiment of the present disclosure.
FIG. 14 is a cross-sectional diagram illustrating a process of radiation therapy of a target site of a living body using a conventional balloon catheter.
FIG. 15 is a cross-sectional view illustrating a non-claimed an MCRT treatment process of a target area of a living body using a balloon catheter according to one embodiment of the present disclosure.
FIG. 16 is a cross-sectional view illustrating an MCRT treatment process of a target area of a living body using a conventional balloon catheter

### [DETAILED DESCRIPTION]

The advantages and features of the present disclosure, and non-claimed methods for achieving them, will become clear with reference to the embodiments described in detail below along with the accompanying drawings. However, the present disclosure is not limited to the embodiments disclosed below and may be implemented in various different forms. The present embodiments are merely provided to ensure that the present disclosure is complete, and provided to fully convey the scope of the present disclosure to those skilled in the art to which the present disclosure pertains. The present disclosure is defined only by the scope of the claims.

The terminology used herein is for the purpose of describing embodiments and is not intended to limit the disclosure. As used herein, singular forms also include plural forms, unless specifically stated otherwise in the context. As used in the specification, "comprises" and/or "comprising" does not exclude the presence or addition of one or more other elements in addition to the mentioned elements. The same reference numerals refer to the same elements throughout the specification, and "and/or" includes each and every combination of one or more of the referenced elements. Although "first", "second", and the like are used to describe various components, these components are of course not limited by these terms. These terms are merely used to distinguish one component from another. Therefore, the first component mentioned below may also be the second component within the technical features of the present disclosure.

Unless otherwise defined, all terms (including technical and scientific terms) used in this specification may be used with meanings commonly understood by those skilled in the art to which this disclosure pertains. Additionally, terms defined in commonly used dictionaries are not to be interpreted ideally or excessively unless clearly specifically defined.

Hereinafter, the embodiments of the present disclosure will be described in detail with reference to the attached drawings.

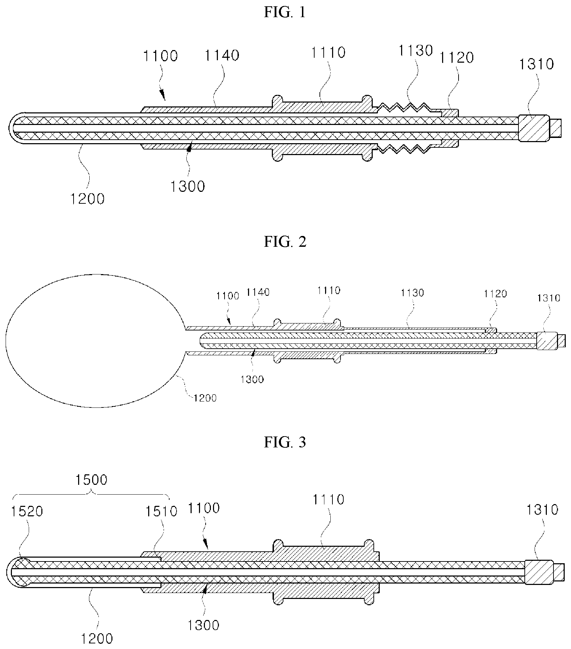

FIG. 1 is a cross-sectional view illustrating a balloon catheter according to one embodiment of the present disclosure, and FIG. 2 is a cross-sectional view illustrating the balloon of FIG. 1 in an inflated state.

As shown in FIG. 1, the balloon catheter according to one embodiment of the present disclosure includes an external catheter 1100, a balloon 1200, an internal catheter 1300, and a driving part.

The external catheter 1100 serves as a basic body of the present disclosure. The external catheter 1100 is open on both sides. Here, one side of the external catheter 1100 is connected by the balloon 1200. In addition, the interior of the external catheter 1100 is may be penetrated by the internal catheter 1300. Furthermore, a handle 1110 for grasping the external catheter 1100 may be formed on the other side of the external catheter 1100. Here, the center of the handle 1110 may have a smaller outer diameter than the two sides of the handle 1110, in other words, the two sides of the handle 1110 may have a multi-stage shape that protrudes from the center of the handle 1110. Accordingly, the two sides of the handle 1110 may serve to prevent the operator's hand grasped at the center of the handle 1110 from being separated in the longitudinal direction of the handle 1110.

As an example, the outer diameter of the external catheter 1100 may be 10 mm to 30 mm, but the present disclosure is not limited thereto. As another example, the length of the external catheter 1100 may be 50 mm to 1,000 mm, but the present disclosure is not limited thereto. As another example, the material of the external catheter 1100 may include at least one of silicone, latex, polyurethane, polyisoprene, and PVC.

The external catheter 1100 includes a coupling part 1120, a stretchable part 1130, and a guide part 1140. Here, the guide part 1140 may be located on one side of the external catheter 1100, the coupling part 1120 may be located on the other side of the external catheter 1100, and the stretchable part 1130 may be located between the one side and the other side of the external catheter 1100.

The coupling part 1120 is coupled to the outer circumference of the internal catheter 1300. As an example, the coupling part 1120 may have a ring shape.

The stretchable part 1130 is connected to one side of the connecting part 1120 and is stretchable in the direction of movement of the internal catheter 1300. For example, the stretchable part 1130 may have a bellows shape.

The guide part 1140 is connected to one side of the stretchable part 1130 and guides the movement of the internal catheter 1300. For example, the guide part 1140 may have a ring shape. Meanwhile, the balloon 1200 may be connected to one side of the guide part 1140.

The balloon 1200 is connected to one side of the external catheter 1100, may be inflated or contracted, and may be inserted into a target site of a living body. This balloon 1200 may be inflated while inserted into the target site of the living body and fixed to a lesion tissue and the target site of the living body. For example, the balloon 1200 may be inflated by fluid injection. For reference, the target site of the living body may be an internal space of the living body that may be inserted without an incision in the living body (e.g., one of the oral cavity, nasal cavity, pharynx, larynx, esophagus, stomach, duodenum, large intestine, and rectum), an internal space of the living body that may be inserted through an incision (e.g., one of the thoracic cavity, abdominal cavity, and inside the skin), but the present disclosure is not limited thereto, and may be applied to other tissues of the living body. In addition, the lesion tissue may be cancer tissue.

For example, the material of the balloon 1200 may include at least one of silicone, latex, polyurethane, and polyisoprene.

The internal catheter 1300 has one side inserted into the inside of the balloon 1200 and the other side penetrated into the inside of the external catheter 1100, and is moved in a first direction inserted into the balloon 1200 or a second direction discharged from the balloon 1200.

For example, when the internal catheter 1300 is moved in the first direction inserted into the balloon 1200, one side of the internal catheter 1300 may be inserted into the inside of the balloon 1200. At this time, the one side of the internal catheter 1300 inserted into the inside of the balloon 1200 may provide rigidity to the balloon 1200 when the balloon 1200 is inserted into the target site of the living body, thereby overcoming the insertion resistance applied to the balloon 1200 in the living body. Thereafter, the balloon 1200 inserted into the target site of the living body may be inflated and fixed to the lesion tissue and target site of the living body. Subsequently, one side of the internal catheter 1300 inserted into the inside of the balloon 1200 is discharged from the inside of the balloon 1200 by the driving part.

As an example, a valve 1310 for sealing the internal catheter may be installed on the other side of the internal catheter 1300. This valve 1310 may serve to allow the inflow of the fluid supplied from a fluid supply part and prevent the outflow of the corresponding fluid.

As an example, the valve 1310 may be composed of at least one of a check valve, a stopcock, and a clamp.

For example, the external catheter 1100 or the internal catheter 1300 may be fixed around the target site of the living body so that the position of the balloon 1200 in the target site of the living body is maintained.

The driving part moves the internal catheter 1300 inserted inside the balloon 1200 in the second direction and discharges the internal catheter 1300 while the balloon 1200 inserted in the target site of the living body is inflated and fixed. This driving part may be operated under the control of a processor.

The driving part may include an actuator and the fluid supply part.

The actuator may move the internal catheter 1300 in the second direction. For example, the actuator may move the internal catheter 1300 in the second direction while the balloon 1200 inserted in the target site of the living body is inflated and fixed under the control of the processor.

The fluid supply part may inject fluid into the balloon 1200 so that the internal catheter 1300 moves in the second direction by the fluid injected into the balloon 1200. Here, the diameter and material of the balloon 1200 may be initially set so that the pressure required for initial inflation is greater than the pressure required for the internal catheter 1300 to move in the second direction. Therefore, in the case that the fluid supply part injects fluid into the interior of the balloon 1200, the gas filled in the interior of the balloon 1200 may push the internal catheter 1300 so that the internal catheter 1300 moves in the second direction.

The fluid supply part may inject fluid through the internal catheter 1300 or the external catheter 1100.

Hereinafter, a process of inserting and fixing a balloon catheter according to one embodiment of the present disclosure into the target site of the living body will be described.

First, the internal catheter 1300 penetrated into the external catheter 1100 is moved in the first direction and inserted into the inside of a balloon 1200.

Subsequently, the balloon 1200 is inserted into the target site of the living body. At this time, one side of the internal catheter 1300 inserted into the inside of the balloon 1200 may provide rigidity to the balloon 1200 when the balloon 1200 is inserted into the target site of the living body, thereby overcoming the insertion resistance applied to the balloon 1200 inside the living body.

Next, the balloon 1200 is inflated and fixed to the lesion tissue and target site of the living body. At this time, the balloon 1200 may be inflated by fluid injection. For example, the fluid injection of the balloon 1200 may be performed through the fluid supply part of the driving part.

Thereafter, the driving part moves the internal catheter 1300 in the second direction so that one side of the internal catheter 1300 is discharged from the inside of the balloon 1200.

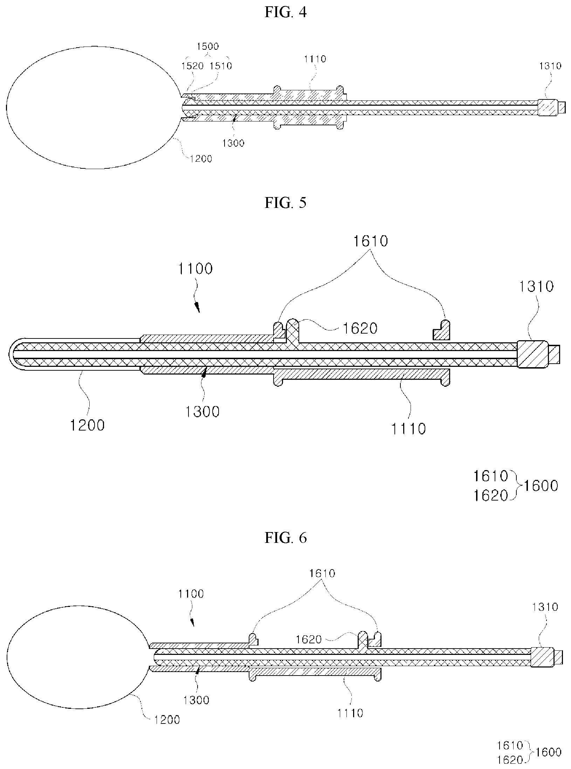

FIG. 3 is a cross-sectional view illustrating a first stopper of a balloon catheter according to an embodiment of the present disclosure, and FIG. 4 is a cross-sectional view illustrating the balloon of FIG. 3 in an inflated state.

As shown in FIG. 3, the balloon catheter according to an embodiment of the present disclosure includes a first stopper 1500, unlike the example of FIG. 1. In this example, the inner circumference of the external catheter 1100 may be closely attached to the outer circumference of the internal catheter 1300. Therefore, air leakage or fluid leakage between the external catheter 1100 and the internal catheter 1300 may be prevented.

The first stopper 1500 serves to limit the movement range of the internal catheter 1300 in the second direction. The first stopper 1500 includes a hook groove 1510 and a hook 1520.

The hook groove 1510 is formed on one side of the external catheter 1100. For example, the hook groove 1510 may be formed by being sunken along the inner circumference of one side of the external catheter 1100. At this time, the hook groove 1510 may have a ring shape.

The hook 1520 is formed in the internal catheter 1300 and is caught in the hook groove 1510 when the internal catheter 1300 moves in the second direction. For example, the hook 1520 may have a shape corresponding to the hook groove 1510.

In this example, when the internal catheter 1300 moves in the second direction, the hook 1520 is caught in the hook groove 1510 and the range of movement of the internal catheter 1300 in the second direction may be limited (Refer to FIG. 4).

FIG. 5 is a cross-sectional view illustrating a second stopper of a balloon catheter according to one embodiment of the present disclosure, and FIG. 6 is a cross-sectional view illustrating the balloon of FIG. 5 in an inflated state.

As illustrated in FIG. 5, the balloon catheter according to one embodiment of the present disclosure includes a second stopper 1600, unlike the example of FIG. 1. In this example, the inner circumference of the external catheter 1100 may be closely attached to the outer circumference of the internal catheter 1300. Therefore, air leakage or fluid leakage through the space between the external catheter 1100 and the internal catheter 1300 may be prevented.

The second stopper 1600 limits the first direction movement range and the second direction movement range of the internal catheter 1300. This second stopper 1600 includes two catch protrusions 1610 and a moving protrusion 1620.

The two catch protrusions 1610 are coupled to the outer circumference of the external catheter 1100 with a gap along the direction of movement of the internal catheter 1300. For example, the two catch protrusions 1610 may have a shape that protrudes vertically from the outer circumference of the external catheter 1100.

The moving protrusion 1620 is coupled to the outer circumference of the internal catheter 1300 and is positioned between the two catch protrusions 1610. For example, the moving protrusion 1620 may have a shape that protrudes vertically from the outer circumference of the internal catheter 1300.

In this example, when the internal catheter 1300 moves in the first direction, the moving protrusion 1620 may be caught by the one located closer to one side of the external catheter 1100 among the two catch protrusions 1610, and the movement range of the internal catheter 1300 in the first direction may be limited (Refer to FIG. 6).

In addition, when the internal catheter 1300 moves in the second direction, the moving protrusion 1620 may be caught by the one located closer to the other side of the external catheter 1100 among the two catch protrusions 1610, and the movement range of the internal catheter 1300 in the second direction may be limited (Refer to FIG. 6).

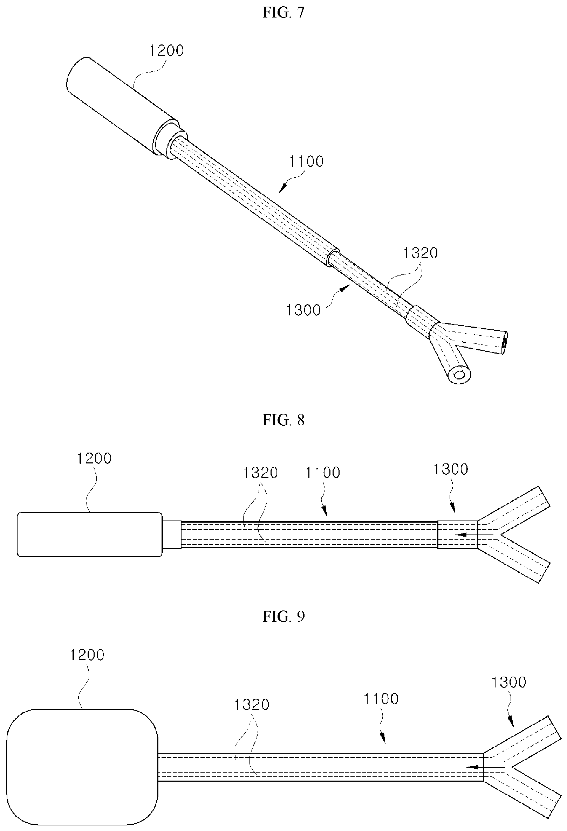

FIGS. 7 to 10 are cross-sectional views illustrating an operation process of a balloon catheter according to another embodiment of the present disclosure.

As shown in FIG. 7, the balloon catheter according to another embodiment of the present disclosure may have the internal catheter 1300 with multiple lumens 1320, unlike the example of FIG. 1, and includes a third stopper 1700.

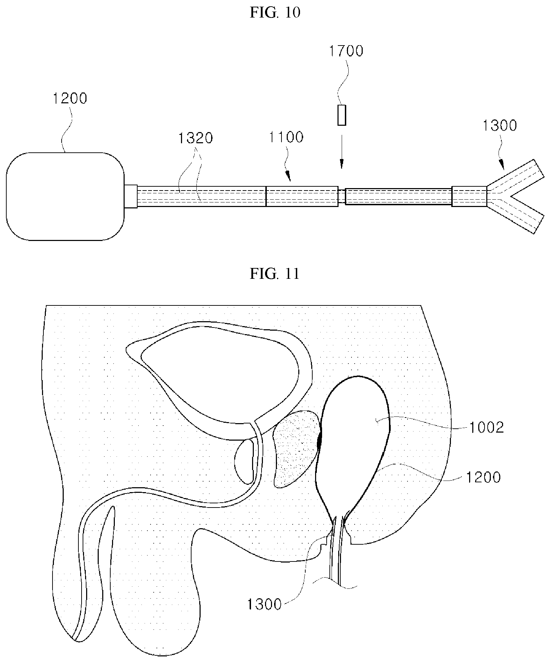

The third stopper 1700 is detachably fixed to the internal catheter 1300 and serves to limit the movement range of the internal catheter 1300 in the first direction (Refer to FIG. 10). As an example, the third stopper 1700 may have a U-shape. This third stopper 1700 is fixed to the outer circumference of the internal catheter 1300 in contact with the other side of the external catheter 1100 while one side of the internal catheter 1300 is discharged from the inside of the balloon 1200, thereby limiting the range of movement of the internal catheter 1300 in the first direction.

Hereinafter, a process of inserting and fixing the balloon catheter according to another embodiment of the present disclosure into a target site of a living body will be described.

First, the internal catheter 1300 penetrated into the external catheter is moved in the first direction and inserted into the inside of the balloon 1200 (Refer to FIG. 8).

Next, the balloon 1200 is inserted into the target site of the living body. At this time, one side of the internal catheter 1300 inserted into the inside of the balloon 1200 may provide rigidity to the balloon 1200 when the balloon 1200 is inserted into the target site of the living body, thereby overcoming the insertion resistance applied to the balloon 1200 inside the living body.

Thereafter, the balloon 1200 is inflated and fixed to the lesion tissue and target site of the living body. At this time, the balloon 1200 may be inflated by fluid injection. For example, the fluid injection of the balloon 1200 may be performed through the fluid supply part of the driving part (Refer to FIG. 9).

Next, the driving part moves the internal catheter 1300 in the second direction so that one side of the internal catheter 1300 is discharged from the inside of the balloon 1200. Thereafter, the third stopper 1700 is fixed to the outer circumference of the internal catheter 1300 that contacts the other side of the external catheter 1100 while one side of the internal catheter 1300 is discharged from the inside of the balloon 1200, thereby limiting the movement range of the internal catheter 1300 in the first direction (Refer to FIG. 10).

As an example, during radiation therapy, one of X-rays, electrons, protons, and carbon particles of 1 MeV or more may be used.

For reference, in the case that there is a balloon with a large or small density inside the living body compared to the target area of the living body, it may take more time to calculate the radiation dose for the radiation treatment plan. Therefore, the balloon 1200 may be set to a predetermined shape and density to shorten the radiation dose calculation time. At this time, the shape of the balloon 1200 may be set to one of a sphere, a hemisphere, an ellipsoid, a cylinder, and a square cylinder. Furthermore, the basic shape of the balloon 1200 may be adjusted when it is ejected, and the expansion shape may be adjusted by adjusting the thickness of each unit area of the balloon 1200. In addition, the internal density of the balloon 1200 may be adjusted by adjusting the composition of the material injected into the balloon 1200.

For example, a sensing module for measuring the volume and pressure of the balloon 1200 may be installed in the external catheter 1100 or the internal catheter 1300, and the sensing module may provide the volume and pressure data of the balloon 1200 to the operator for monitoring the volume and pressure of the balloon 1200.

For example, the internal temperature of the balloon 1200 may be controlled through a fluid circulation device that circulates the fluid injected into two or more lumens 1320 of the internal catheter 1300. At this time, the temperature of the balloon 1200 may affect the therapeutic effect or side effect of the target area of the living body that comes into contact with the balloon 1200, and the higher the temperature of the balloon 1200, the greater the effect and side effect due to radiation, and the lower the temperature of the balloon 1200, the less the effect and side effect due to radiation.

For example, the balloon 1200 may be coated with a radiation-sensitive material that changes color when exposed to radiation. Accordingly, when the balloon 1200 is exposed to radiation, the color of the balloon 1200 may change, and through this, the radiation dose irradiated and absorbed by the balloon 1200 may be calculated, and through this, the radiation dose irradiated and absorbed by the target area of the living body in contact with the balloon 1200 may be calculated. Meanwhile, the color change of the balloon 1200 may be identified in real time through an endoscope inserted into the target area of the living body, or may be identified through the balloon 1200 discharged from the target area of the living body after the end of radiation treatment.

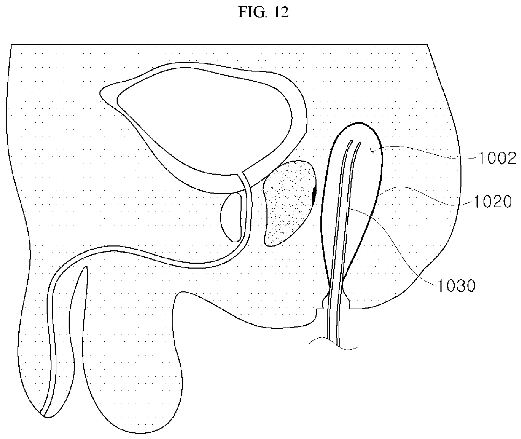

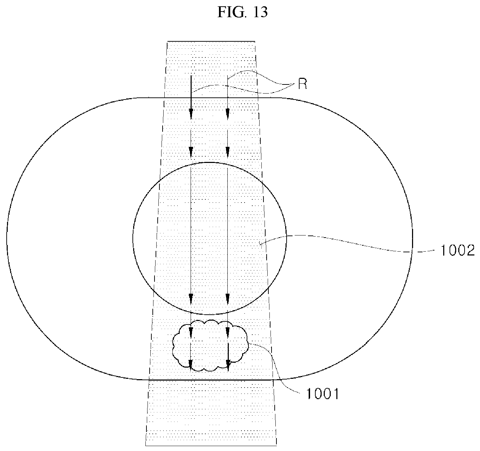

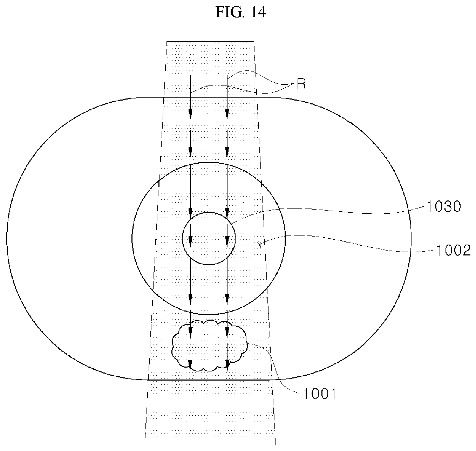

FIG. 11 is a schematic diagram illustrating a state in which a balloon of a balloon catheter according to one embodiment of the present disclosure is fixed to a target site of a living body, FIG. 12 is a schematic diagram illustrating a state in which a balloon of a conventional balloon catheter is fixed to a target site of a living body, FIG. 13 is a cross-sectional diagram illustrating a process of radiation therapy of a target site of a living body using a balloon catheter according to one embodiment of the present disclosure, and FIG. 14 is a cross-sectional diagram illustrating a process of radiation therapy of a target site of a living body using a conventional balloon catheter.

Referring to FIG. 11, in the state that the balloon 1200 of the balloon catheter according to one embodiment of the present disclosure is fixed to a target site 1002 of the living body, the internal catheter 1300 does not exist inside the balloon 1200 fixed to the target site 1002 of the living body. Therefore, during radiation therapy, it may be identified that the radiation R penetrating the inside of the balloon 1200 reaches the lesion tissue 1 of the living body without being affected by the internal catheter 1300 (Refer to FIG. 13).

On the other hand, referring to FIG. 12, in the state that the balloon 1020 of the conventional balloon catheter is fixed to the target area of the living body, it may be identified that the internal catheter 1030 exists inside the balloon 1020 fixed to the target area 1002 of the living body. Therefore, during radiation therapy, it may be identified that the radiation R penetrating the inside of the balloon 1200 is affected by the internal catheter 1300 and reaches the lesion tissue 1 of the living body (Refer to FIG. 14). Due to this, the accuracy of radiation therapy in the conventional technology may be reduced.

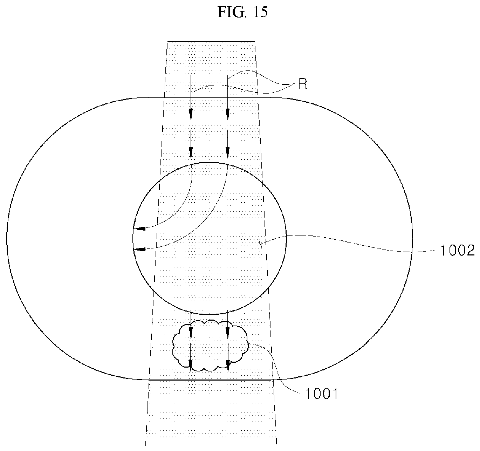

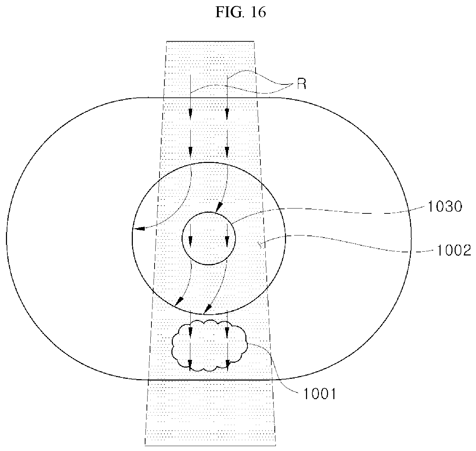

FIG. 15 is a cross-sectional view illustrating a non-claimed MCRT treatment process of a target area of a living body using a balloon catheter according to one embodiment of the present disclosure, and FIG. 16 is a cross-sectional view illustrating an MCRT treatment process of a target area of a living body using a conventional balloon catheter.

Referring to FIG. 15, in the balloon catheter according to one embodiment of the present disclosure, there is no internal catheter 1300 inside the balloon 1200 fixed to the target area 1002 of the living body. Accordingly, in MCRT (Magnetic Controlled Radiation Therapy) that controls the path of radiation using a magnetic field, since there is no internal catheter 1300 on the path of radiation penetrating the inside of the balloon 1200, the generation of secondary electrons due to the influence of the internal catheter 1300 on the radiation may be prevented.

On the other hand, referring to FIG. 16, the conventional balloon catheter has an internal catheter 1300 inside the balloon 1200 fixed to the target site 1002 of the living body. Therefore, during MCRT, which controls the path of radiation using a magnetic field, since the internal catheter 1030 is on the path of radiation penetrating the inside of the balloon 1020, secondary electrons are generated due to the influence of the internal catheter 1030 on the radiation. Due to this, the conventional technology has a reduced accuracy in controlling the path of radiation by MCRT.

Therefore, the balloon catheter according to one embodiment of the present disclosure has the effect of improving the accuracy of radiation treatment for the lesion tissue and target site of the living body during radiation treatment by enabling the discharge of the internal catheter inserted inside the balloon while the balloon is inflated and fixing the lesion tissue and target site of the living body.

Although the embodiments of the present disclosure have been described above with reference to the attached drawings, those skilled in the art will understand that the present disclosure may be implemented in other specific forms without changing the technical idea or essential features thereof. Therefore, it should be understood that the embodiments described above are exemplary in all respects and not restrictive.

## Claims

1. A balloon catheter comprising:
an external catheter; (1100)
a balloon (1200) connected to one side of the external catheter and capable of being inflated or contracted and inserted into a target site of a living body;
an internal catheter (1300) having one side inserted into the balloon and the other side penetrating into an inside of the external catheter and capable of moving in a first direction to be inserted into the balloon and a second direction to be discharged from the balloon;
a driving part configured to move and discharge the internal catheter inserted into the inside of the balloon in the second direction in a state that the balloon is inflated and fixed to the target site of the living body; and
a first stopper (1500) configured to limit a movement range of the internal catheter in the second direction,
**characterized in that** wherein the first stopper comprises:
a hook groove (1510) formed in the external catheter; and
a hook (1520) formed in the internal catheter and hooked to the hook groove when the internal catheter moves in the second direction.

2. The balloon catheter of claim 1, wherein the driving part comprises:
an actuator configured to move the internal catheter (1300) in the second direction.

3. A balloon catheter comprising:
an external catheter (1100);
a balloon (1200) connected to one side of the external catheter and capable of being inflated or contracted and inserted into a target site of a living body;
an internal catheter (1300) having one side inserted into the balloon and the other side penetrating into an inside of the external catheter and capable of moving in a first direction to be inserted into the balloon and a second direction to be discharged from the balloon; and
a driving part configured to move and discharge the internal catheter inserted into the inside of the balloon in the second direction in a state that the balloon is inflated and fixed to the target site of the living body,
**characterized in that** the external catheter comprises:
a coupling part (1120) coupled to an outer circumference of the internal catheter;
a stretchable part (1130) coupled to the coupling part and capable of being stretched; and
a guide part (1140) coupled to the stretchable part and configured to guide a movement of the internal catheter, and wherein the balloon is connected to the guide part.

4. The balloon catheter of claim 3, wherein the stretchable part (1130) has a bellows shape.

5. A balloon catheter comprising:
an external catheter; (1100)
a balloon (1200) connected to one side of the external catheter and capable of being inflated or contracted and inserted into a target site of a living body;
an internal catheter (1300) having one side inserted into the balloon and the other side penetrating into an inside of the external catheter and capable of moving in a first direction to be inserted into the balloon and a second direction to be discharged from the balloon;
a driving part configured to move and discharge the internal catheter inserted into the inside of the balloon in the second direction in a state that the balloon is inflated and fixed to the target site of the living body; and
a second stopper (1600) configured to limit a movement range of the internal catheter in the first direction and a movement range of the second direction,
**characterized in that** the second stopper comprises:
two catch protrusions (1610) spaced apart from each other and connected along a movement direction of the internal catheter to the outer circumference of the external catheter; and
a moving protrusion (1620) connected to the outer circumference of the internal catheter and is arranged between the two catch protrusions.

6. A balloon catheter comprising:
an external catheter; (1100)
a balloon (1200) connected to one side of the external catheter and capable of being inflated or contracted and inserted into a target site of a living body;
an internal catheter (1300) having one side inserted into the balloon and the other side penetrating into an inside of the external catheter and capable of moving in a first direction inserted into the balloon and a second direction to be discharged from the balloon; and
a driving part configured to move and discharge the internal catheter to be inserted into the inside of the balloon in the second direction in a state that the balloon is inflated and fixed to the target site of the living body,
**characterized in that** the balloon catheter further comprises a third stopper (1700) detachably fixed to the internal catheter and configured to limit a movement range of the internal catheter in the first direction.

7. The balloon catheter of claim 6, wherein the third stopper (1700) has a U-shape.

## Patentansprüche

1. Ballonkatheter, umfassend:
einen externen Katheter; (1100)
einen Ballon (1200), der mit einer Seite des externen Katheters verbunden und in der Lage ist, aufgeblasen oder kontrahiert und in eine Zielstelle eines lebenden Körpers eingeführt zu werden;
einen internen Katheter (1300) mit einer Seite, die in den Ballon eingeführt ist, und der anderen Seite, die in ein Inneres des externen Katheters eindringt und in der Lage ist, sich in einer ersten Richtung, um in den Ballon eingeführt zu werden, und einer zweiten Richtung, um aus dem Ballon ausgestoßen zu werden, zu bewegen;
ein Antriebsteil, das dazu konfiguriert ist, den internen Katheter, der in das Innere des Ballons eingeführt ist, in der zweiten Richtung in einem Zustand zu bewegen und auszustoßen, in dem der Ballon aufgeblasen und an der Zielstelle des lebenden Körpers fixiert ist; und
einen ersten Anschlag (1500), der dazu konfiguriert ist, eine Bewegungsspanne des internen Katheters in der zweiten Richtung zu begrenzen,
**dadurch gekennzeichnet, dass** wobei der erste Anschlag Folgendes umfasst:
eine Hakennut (1510), die in dem externen Katheter gebildet ist; und
einen Haken (1520), der in dem internen Katheter gebildet und an der Hakennut eingehakt ist, wenn sich der interne Katheter in der zweiten Richtung bewegt.

2. Ballonkatheter nach Anspruch 1, wobei das Antriebsteil Folgendes umfasst:
einen Aktor, der dazu konfiguriert ist, den internen Katheter (1300) in der zweiten Richtung zu bewegen.

3. Ballonkatheter, umfassend:
einen externen Katheter (1100);
einen Ballon (1200), der mit einer Seite des externen Katheters verbunden und in der Lage ist, aufgeblasen oder kontrahiert und in eine Zielstelle eines lebenden Körpers eingeführt zu werden;
einen internen Katheter (1300) mit einer Seite, die in den Ballon eingeführt ist, und der anderen Seite, die in ein Inneres des externen Katheters eindringt und in der Lage ist, sich in einer ersten Richtung, um in den Ballon eingeführt zu werden, und einer zweiten Richtung, um aus dem Ballon ausgestoßen zu werden, zu bewegen; und
ein Antriebsteil, das dazu konfiguriert ist, den internen Katheter, der in das Innere des Ballons eingeführt ist, in der zweiten Richtung in einem Zustand zu bewegen und auszustoßen, in dem der Ballon aufgeblasen und an der Zielstelle des lebenden Körpers fixiert ist,
**dadurch gekennzeichnet, dass** der externe Katheter Folgendes umfasst:
ein Kopplungsteil (1120), das an einen Außenumfang des internen Katheters gekoppelt ist;
ein dehnbares Teil (1130), das an das Kopplungsteil gekoppelt und in der Lage ist, gedehnt zu werden; und
ein Führungsteil (1140), das an das dehnbare Teil gekoppelt und dazu konfiguriert ist, eine Bewegung des internen Katheters zu führen, und wobei der Ballon mit dem Führungsteil verbunden ist.

4. Ballonkatheter nach Anspruch 3, wobei das dehnbare Teil (1130) eine Balgform aufweist.

5. Ballonkatheter, umfassend:
einen externen Katheter; (1100)
einen Ballon (1200), der mit einer Seite des externen Katheters verbunden und in der Lage ist, aufgeblasen oder kontrahiert und in eine Zielstelle eines lebenden Körpers eingeführt zu werden;
einen internen Katheter (1300) mit einer Seite, die in den Ballon eingeführt ist, und der anderen Seite, die in ein Inneres des externen Katheters eindringt und in der Lage ist, sich in einer ersten Richtung, um in den Ballon eingeführt zu werden, und einer zweiten Richtung, um aus dem Ballon ausgestoßen zu werden, zu bewegen;
ein Antriebsteil, das dazu konfiguriert ist, den internen Katheter, der in das Innere des Ballons eingeführt ist, in der zweiten Richtung in einem Zustand zu bewegen und auszustoßen, in dem der Ballon aufgeblasen und an der Zielstelle des lebenden Körpers fixiert ist; und
einen zweiten Anschlag (1600), der dazu konfiguriert ist, eine Bewegungsspanne des internen Katheters in der ersten Richtung und eine Bewegungsspanne der zweiten Richtung zu begrenzen,
**dadurch gekennzeichnet, dass** der zweite Anschlag Folgendes umfasst:
zwei Rastvorsprünge (1610), die voneinander beabstandet und entlang einer Bewegungsrichtung des internen Katheters mit dem Außenumfang des externen Katheters verbunden sind; und
einen Bewegungsvorsprung (1620), der mit dem Außenumfang des internen Katheters verbunden ist und zwischen den zwei Rastvorsprüngen angeordnet ist.

6. Ballonkatheter, umfassend:
einen externen Katheter; (1100)
einen Ballon (1200), der mit einer Seite des externen Katheters verbunden und in der Lage ist, aufgeblasen oder kontrahiert und in eine Zielstelle eines lebenden Körpers eingeführt zu werden;
einen internen Katheter (1300) mit einer Seite, die in den Ballon eingeführt ist, und der anderen Seite, die in ein Inneres des externen Katheters eindringt und in der Lage ist, sich in einer ersten Richtung, die in den Ballon eingeführt ist, und einer zweiten Richtung, um aus dem Ballon ausgestoßen zu werden, zu bewegen; und
ein Antriebsteil, das dazu konfiguriert ist, den internen Katheter, der in das Innere des Ballons einzuführen ist, in der zweiten Richtung in einem Zustand zu bewegen und auszustoßen, in dem der Ballon aufgeblasen und an der Zielstelle des lebenden Körpers fixiert ist,
**dadurch gekennzeichnet, dass** der Ballonkatheter ferner einen dritten Anschlag (1700) umfasst, der lösbar an dem internen Katheter fixiert und dazu konfiguriert ist, eine Bewegungsspanne des internen Katheters in der ersten Richtung zu begrenzen.

7. Ballonkatheter nach Anspruch 6, wobei der dritte Anschlag (1700) eine U-Form aufweist.

## Revendications

1. Cathéter à ballonnet comprenant :
un cathéter externe ; (1100)
un ballonnet (1200) relié à un côté du cathéter externe et pouvant être gonflé ou contracté et inséré dans un site cible d'un corps vivant ;
un cathéter interne (1300) ayant un côté inséré dans le ballonnet et l'autre côté pénétrant à l'intérieur du cathéter externe et pouvant se déplacer dans un premier sens de manière à être inséré dans le ballonnet et dans un second sens de manière à être déchargé du ballonnet ;
une pièce d'entraînement conçue pour déplacer et décharger le cathéter interne inséré à l'intérieur du ballonnet dans le second sens dans un état dans lequel le ballonnet est gonflé et fixé au site cible du corps vivant ; et
une première butée (1500) conçue pour limiter une plage de déplacement du cathéter interne dans le second sens,
**caractérisé en ce que** ladite première butée comprend :
une rainure de crochet (1510) formée dans le cathéter externe ; et
un crochet (1520) formé dans le cathéter interne et accroché à la rainure de crochet lorsque le cathéter interne se déplace dans le second sens.

2. Cathéter à ballonnet selon la revendication 1, ladite pièce d'entraînement comprenant :
un actionneur conçu pour déplacer le cathéter interne (1300) dans le second sens.

3. Cathéter à ballonnet comprenant :
un cathéter externe (1100) ;
un ballonnet (1200) relié à un côté du cathéter externe et pouvant être gonflé ou contracté et inséré dans un site cible d'un corps vivant ;
un cathéter interne (1300) ayant un côté inséré dans le ballonnet et l'autre côté pénétrant à un intérieur du cathéter externe et pouvant se déplacer dans un premier sens de manière à être inséré dans le ballonnet et dans un second sens de manière à être déchargé du ballonnet ; et
une pièce d'entraînement conçue pour déplacer et décharger le cathéter interne inséré à l'intérieur du ballonnet dans le second sens dans un état dans lequel le ballonnet est gonflé et fixé au site cible du corps vivant,
**caractérisé en ce que** le cathéter externe comprend :
une pièce de couplage (1120) couplée à la circonférence externe du cathéter interne ;
une pièce étirable (1130) couplée à la pièce de couplage et pouvant être étirée ; et
une pièce de guidage (1140) couplée à la pièce étirable et conçue pour guider un déplacement du cathéter interne, et ledit ballonnet étant relié à la pièce de guidage.

4. Cathéter à ballonnet selon la revendication 3, ladite pièce étirable (1130) présentant une forme de soufflet.

5. Cathéter à ballonnet comprenant :
un cathéter externe ; (1100)
un ballonnet (1200) relié à un côté du cathéter externe et pouvant être gonflé ou contracté et inséré dans un site cible d'un corps vivant ;
un cathéter interne (1300) ayant un côté inséré dans le ballonnet et l'autre côté pénétrant à l'intérieur du cathéter externe et pouvant se déplacer dans un premier sens de manière à être inséré dans le ballonnet et dans un second sens de manière à être déchargé du ballonnet ;
une pièce d'entraînement conçue pour déplacer et décharger le cathéter interne inséré à l'intérieur du ballonnet dans le second sens dans un état dans lequel le ballonnet est gonflé et fixé au site cible du corps vivant ; et
une seconde butée (1600) conçue pour limiter une plage de déplacement du cathéter interne dans le premier sens et une plage de déplacement du second sens,
**caractérisé en ce que** la seconde butée comprend :
deux saillies de verrouillage (1610) espacées l'une de l'autre et reliées le long d'un sens de déplacement du cathéter interne à la circonférence externe du cathéter externe ; et
une saillie mobile (1620) reliée à la circonférence externe du cathéter interne et disposée entre les deux saillies de verrouillage.

6. Cathéter à ballonnet comprenant :
un cathéter externe ; (1100)
un ballonnet (1200) relié à un côté du cathéter externe et pouvant être gonflé ou contracté et inséré dans un site cible d'un corps vivant ;
un cathéter interne (1300) ayant un côté inséré dans le ballonnet et l'autre côté pénétrant à un intérieur du cathéter externe et pouvant se déplacer dans un premier sens inséré dans le ballonnet et dans un second sens de manière à être déchargé du ballonnet ; et
une pièce d'entraînement conçue pour déplacer et décharger le cathéter interne de manière à être inséré à l'intérieur du ballonnet dans le second sens dans un état dans lequel le ballonnet est gonflé et fixé au site cible du corps vivant,
**caractérisé en ce que** le cathéter à ballonnet comprend en outre une troisième butée (1700) fixée de manière amovible au cathéter interne et conçu pour limiter une plage de déplacement du cathéter interne dans le premier sens.

7. Cathéter à ballonnet selon la revendication 6, ladite troisième butée (1700) présentant une forme en U.
